# EUROPEAN PATENT APPLICATION

(11) **EP 4 663 305 A1**
(43) Date of publication of application: **17.12.2025**
(21) Application number: 25182686.3
(22) Date of filing: 13.06.2025
(51) Int. Cl.: B05B 1/00, A61M 15/00, B05B 11/10, B05B 12/08, G01M 3/18

(54) **A METHOD AND APPARATUS FOR TESTING THE FUNCTIONALITY OF A LIQUID DELIVERY DEVICE**

(30) Priority: 14.06.2024 GB 202408551
(71) Applicant: Merxin Ltd, King's Lynn, Norfolk PE30 5FQ (GB)
(72) Inventor: Purkins, Graham, King's Lynn, PE32 1EN (GB); Stuart, Adam, King's Lynn, PE32 1BS (GB); Antoniak, Dylan, King's Lynn, PE30 3UW (GB)
(74) Representative: Basck Limited

(57) **Abstract**

A method for testing the functionality of a liquid delivery device comprises the steps of:
(i) fluidically connecting a liquid delivery device (or part thereof) to a pumping system;
(ii) connecting the pumping system to a fluid reservoir;
(iii) running the pumping system so that fluid from the reservoir is forced through the liquid delivery device (or part thereof) under pressure;
(iv) measuring the pressure of the fluid as the fluid passes into the liquid delivery device (or part thereof);
(v) comparing the plotted pressure with the test results for a nominal or within specification liquid delivery device (or part thereof).

## Description

### TECHNICAL FIELD

The present invention relates to a method of testing the functionality of a liquid delivery device. The present invention also relates to an apparatus for testing the functionality of a liquid delivery device.

### BACKGROUND

Drug delivery devices such as nebulisers are used to produce an aerosol of droplets for inhalation into the lungs of a patient through the mouth and pharyngeal cavity, for nasal administration, or for spraying the surface of the eye. A drug delivery device of this type is a particular form of liquid delivery device.

In a nebulising drug delivery device such as a soft mist inhaler (SMI), liquid pharmaceutical formulations are typically stored within the drug delivery device in a reservoir located in the lower part of the casing of the SMI. In a typical known type of SMI, the liquid pharmaceutical formulations are conveyed from the reservoir, through a riser tube running within the SMI from the lower part to the upper part, and into a pressure chamber located in the upper part. The liquid formulation is then forced through a nozzle under pressure and atomised. In this way, drug delivery devices such as SMIs are able to nebulise a small amount of a liquid formulation within a few seconds, in order to produce a required dosage in aerosol form suitable for therapeutic inhalation. Moreover, this can be achieved without requiring the use of a separate propellant.

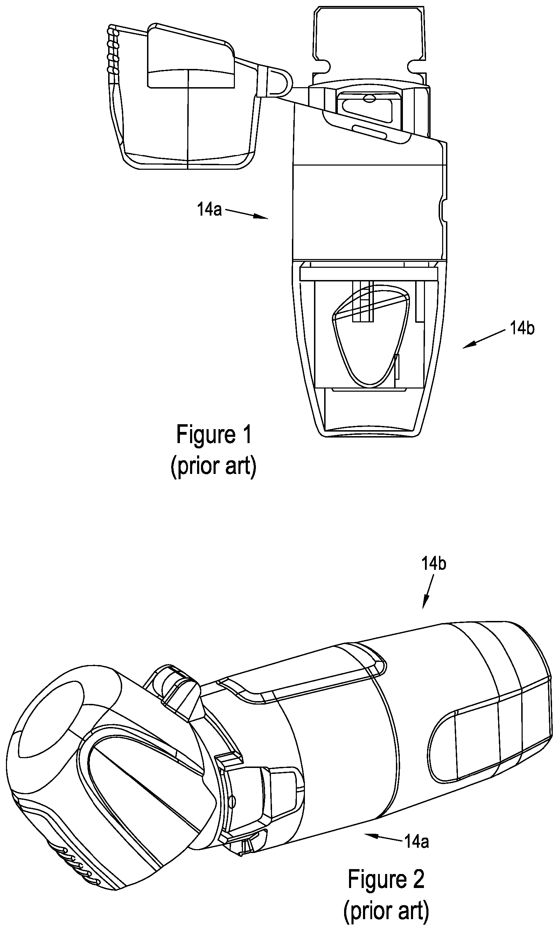

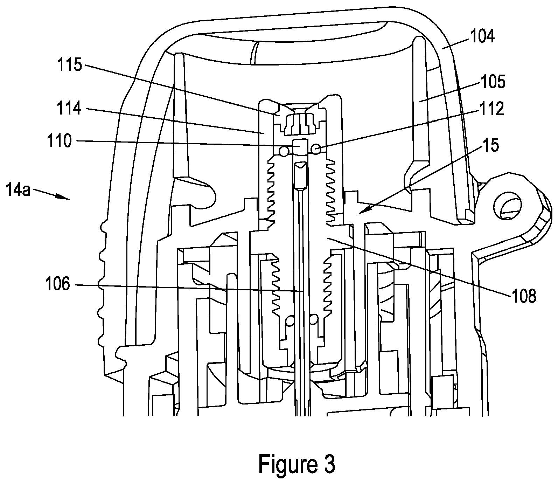

A typical known type of SMI or nebuliser device is shown in figures 1 to 3. The device has an upper half that contains a nozzle and mouthpiece, and a lower half that in use contains a reservoir of liquid. A riser tube extends axially along the device from the reservoir to the nozzle. In use, the upper and lower halves are rotated relative to one another to pump or prime the device. When the device is subsequently triggered by a user, liquid is forced through the nozzle under pressure so that it becomes a nebulised mist that is delivered to a user.

An important concern when manufacturing devices of this type is to ensure that the nebulised mist is delivered as intended. A functional nebuliser for generating an inhalable aerosol should deliver a defined quantity of the fluid or drug formulation in the form of an aerosol cloud or spray mist, the mist itself having defined properties. However, if there is a blockage or leaks between the reservoir and the exit of the nozzle then when the device is triggered this can change several of the delivery parameters, such as for example one or more of: the droplet size, the delivery pressure, the pressure profile, the plume shape, the dose size, and the delivery duration. If for example the nebuliser is being used to deliver medicine for inhalation by a user, a change in the delivery pressure, and/or pressure profile, and/or the droplet size, can mean that the delivered dose of medicament is less effective than would otherwise be the case.

Functionality can be checked using measured values that are determined during actuation of the device, and then comparing these to target values and/or limit values. However, conducting functionality checks on devices on an assembly line can be costly, especially if multiple different parameters are required to be measured. 100% inspection or fully testing every device is frequently not practical. Random inspections are often used in order to check overall or average manufacturing quality, but sub-standard devices may still be missed.

The basic functionality of a device can be checked by measuring the generated aerosol cloud or the generated spray, for example by direct measurements of droplet size distributions (e.g. using optical methods or cascade impactors), measurements of the total mass of the fluid released in the spray, etc. WO2017/060328 describes and shows a system and associated method for testing the functionality of an atomiser for dispensing a fluid in the form of an aerosol. The system contains an atomiser to be tested, and a testing device. In use, the container moves relative to the housing part in order to dispense the fluid, and the testing device incorporates a measuring device for measuring the movement of the container when dispensing the fluid. The movement of the container is measured and/or analysed when dispensing the fluid.

In this specification where reference has been made to patent specifications, other external documents, or other sources of information, this is generally for the purpose of providing a context for discussing the features of the invention. Unless specifically stated otherwise, reference to such external documents is not to be construed as an admission that such documents, or such sources of information, in any jurisdiction, are prior art, or form part of the common general knowledge in the art.

### SUMMARY OF THE INVENTION

It is an object of the present invention to provide a method for testing the functionality of a liquid delivery device which goes some way to overcoming the abovementioned disadvantages or which at least provides the public or industry with a useful choice. It is a further object of the present invention to provide an apparatus for testing the functionality of a liquid delivery device which goes some way to overcoming the abovementioned disadvantages or which at least provides the public or industry with a useful choice.

Accordingly, in a first aspect the present invention may broadly be said to consist in a **method for testing the functionality of a liquid delivery device,** comprising the steps of:
(i) fluidically connecting a liquid delivery device (or part thereof) to a pumping system;
(ii) connecting the pumping system to a fluid reservoir;
(iii) running the pumping system so that fluid from the reservoir is forced through the liquid delivery device (or part thereof) under pressure;
(iv) measuring the pressure of the fluid as the fluid passes into the liquid delivery device (or part thereof);
(v) comparing the plotted pressure with the test results for a nominal or within specification liquid delivery device (or part thereof).

In an embodiment, in the step of connecting the pump to a fluid reservoir, the pump is connected to a reservoir containing ethanol.

In an embodiment, in the step of running the pump, the pump is run so as to deliver fluid at a rate of substantially up to 1.1 mL/min through the liquid delivery device.

In an embodiment, in the step of running the pump, the pump is run so as to deliver fluid at a rate of substantially 0.7 mL/min through the liquid delivery device.

In a second aspect the present invention may broadly be said to consist in an **apparatus for testing the functionality of a liquid delivery device,** comprising: a high pressure pumping system; at least one pressure sensor connected the pumping system and configured to measure the pressure or resistance of the liquid delivery device; a computer adapted to connect to and control the pump, and to receive data from at least one pressure sensor and to plot the measured pressure against time.

This invention may also be said broadly to consist in the parts, elements and features referred to or indicated in the specification of the application, individually or collectively, and any or all combinations of any two or more said parts, elements or features, and where specific integers are mentioned herein which have known equivalents in the art to which this invention relates, such known equivalents are deemed to be incorporated herein as if individually set forth.

Therefore, the foregoing is considered as illustrative only of the principles of the invention. Further, since numerous modifications and changes will readily occur to those skilled in the art, it is not desired to limit the invention to the exact construction and operation shown and described, and accordingly, all suitable modifications and equivalents may be resorted to, falling within the scope of the invention.

### BRIEF DESCRIPTION OF THE DRAWINGS

One or more embodiments of the invention will now be described, by way of example only, with reference to the accompanying drawings, in which:
**Figure 1** shows a side view of a known, prior art type of manually-operated nebuliser that has an upper housing part and a lower housing part , the two housing parts rotated relative to one another in use to pump or prime the device for use.
**Figure 2** shows a perspective view from one side and slightly above of a known, prior art nebuliser similar to that of figure 1, the nebuliser having a lid that is shown slightly open.
**Figure 3** shows a perspective cutaway side view of view of the upper housing part of the known, prior art nebuliser of figures 1 and 2, showing internal detail of the upper housing part, the upper housing part comprising a pump core.
**Figure 4a** shows a cutaway schematic side view of the pump core of the prior art nebuliser of figure 3, the pump core comprising an upper tube housing, a top nut, a filter holder, a pre-filter and chip/filter, a nozzle retainer, and upper and lower seals, the upper tube housing comprising a central or axial passage configured to in use hold a piston.
**Figure 4b** shows the pump core with a piston located in the central passage.
**Figure 5** shows a simplified schematic overview of an embodiment of the testing system of the present invention, the testing system comprising a high pressure pumping system, a pressure sensor or sensors connected the pumping system to measure the pressure or resistance of the liquid delivery device, a power pack to power the pressure sensor(s), a computer adapted to connect to and control the pump and to receive data from the pressure sensor(s) via a data capture device, with the pump receiving fluid from a reservoir, and the delivery device to be tested connected to a fixture.
**Figure 6** shows an example graph of pressure/voltage (shown on the y-axis) plotted against time (shown on the x-axis), for six test runs of the core of an inhaler device, with a flow rate of 0.7 mL/min.
**Figure 7** shows an example graph of pressure/voltage (shown on the y-axis) plotted against time (shown on the x-axis), for the test results for a nominal or within specification core, a wide-channel pump core, and a narrow-channel pump core.
**Figure 8** shows a graph with pressure plotted on the y-axis against time on the x-axis, showing the results of two separate test runs carried out on the same pump core, the pump core used in this test of the type that has two outlets, with one plot showing a first test run carried out on the core, and the other plot showing the second test run carried out on the core.
**Figure 9** shows a graph with pressure plotted on the y-axis against time on the x-axis, showing the results of separate test runs carried out on separate cores, one core having a damaged seal, one core having a partially blocked nozzle, one core with an inverted nozzle, and one that has a damaged component that causes a pressure leak, with a 'standard' or nominal plot for a core operating within specification also shown for comparison purposes.
**Figure 10** shows part of an embodiment of a fixture suitable for testing pump cores, the fixture fluidically connected to a pump via hydraulic tubing/connectors, the figure showing a three-way union to allow a pressure sensor to be connected in-line.

### DETAILED DESCRIPTION

Detailed embodiments of the invention will now be described with reference to the figures.

### GENERAL

It has been found that a good approximation of operability performance can be made by measuring the pressure drop in a component (the pressure drop being the difference in pressure between the upstream side and the downstream side of a component).

The pressure measurement can by itself give an indication of operability performance. However, combining this with the flow rate (how much fluid is flowing through the system, measured in appropriate volume per time units such as for example litres/second, etc.) can be used to provide the 'resistance' of a system. The system's 'resistance' provides an indication of the amount or level of pressure required to achieve a particular 'unit' of flowrate, or an indication of how much pressure is required to maintain a certain flowrate through the system.

This can be useful for designing systems, comparing the performance of different devices, or troubleshooting issues like blockages or leaks.

The hydraulic resistance or fluid dynamic resistance can be calculated from the pressure and flow rate, for fluid flowing through a nebuliser/SMI 'pump core'. A fluid such as ethanol is pumped through the core, and at least the pressure is measured, and also optionally but preferably the flow rate. Ethanol is preferred for the fluid, but other fluids can also be used. This test has a quick cycle time, and requires less management of fluid/aerosolised matter due the test not requiring a firing chamber to measure and characterise spray quality. The equipment required is relatively low cost, and the test as a whole is less costly to implement than other types of known tests.

As shown in figures 1 and 2, a typical known type of SMI comprises an upper housing part 14a and a lower housing part 14b. The upper housing part 14a has a mouthpiece 105 and lid 104, and contains a core (generally designated as 'core 15') that forms a part of a nozzle assembly. The lower housing part 14b contains a cartridge that forms a liquid reservoir for the device.

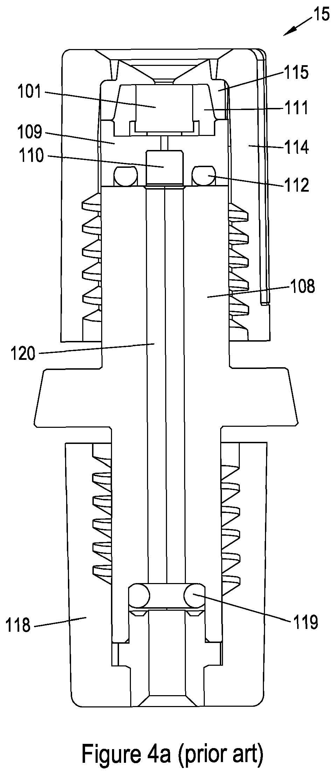

As shown in figure 4a, the core 15 comprises the following main parts: an upper tube housing 108, a filter holder 109; a pre-filter 110; an upper seal or nozzle seal 111; a lower seal 112; a chip/filter 101; a top nut 114; a nozzle retainer 115; a bottom nut 118, and; a capillary O-ring 119.

The filter holder 109, pre-filter 110, upper and lower seals 111, 112, nozzle retainer 115 and the chip/filter 101 are contained within the top nut 114. The top nut 114 is screwed to the top of the upper tube housing, the top nut 114 and upper tube housing 108 mutually threaded to allow them to be screwed together. A central or axial passage 120 runs the length of the upper tube housing 108.

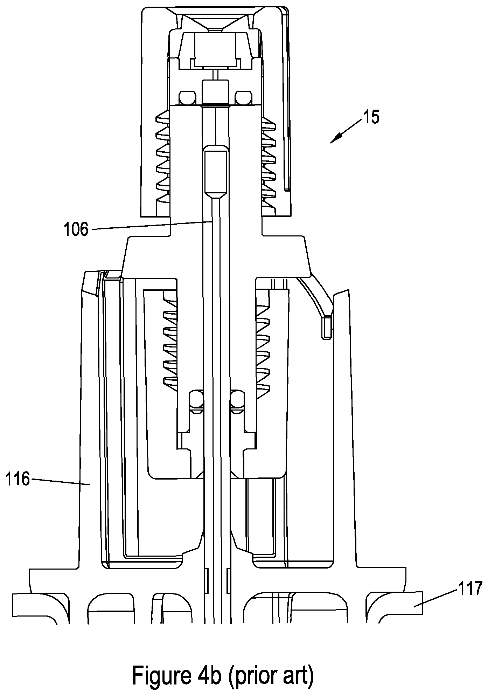

In use, and as shown in figure 4b (with the core 15 connected to the drive component 116 and spring cap 117 as it would be when in use), a capillary tube 106 is located in the central passage 120 of the upper tube housing 108. In use, liquid from the cartridge is sucked up the tube 106, and delivered through the mouthpiece via the nozzle assembly.

### TESTING APPARATUS

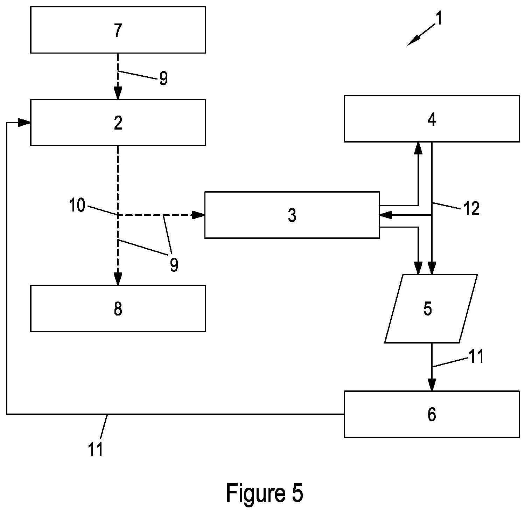

A simplified overview schematic of an embodiment of the testing system/apparatus 1 used for this type of test is shown in figure 5.

The testing apparatus 1 comprises a high pressure pumping system 2, and a pressure sensor or sensors 3 connected to the pumping system 2 to measure the pressure or resistance of the 'pump core'. A power pack 4 is provided to power the pressure sensor(s) 3. A computer 6 is adapted to connect to and control the pump 2, and to receive data from the pressure sensor(s) 3 via a data capture device 5. The pump 2 receives fluid from a reservoir 7.

In use, a pump core 15 to be tested is connected to a fixture 8 that is fluidically connected to the pump 2. In the preferred embodiment, the pump 2 pumps a liquid at high pressure through the core 15 at a given flow rate, and the response from the pressure sensor(s) 3 is used to determine the functionality of the core. However, it should be noted that other fixed parameters could also be used in place of flow rate, such as for example piston delivery or pressure delivery (that is, an input parameter comprising for example a piston of known size, travelling a known distance, with either a known force or time between points, could be used to provide the input flow or pressure). It should also be noted that the pump core 15 could also be tested with the capillary tube 106 present.

Fluidic connections are made between the pump 2, the pressure sensor 3, the reservoir 7, and the fixture 8, via hydraulic tubing/connectors 9.

### High Pressure Pumping System

In this embodiment, the pump 6 comprises a Knauer Azura 6.1L isocratic pump, which can produce a flow of 0.001-10 mL/min, with a maximum pressure of 862 bar.

The standard flowrate used in testing is 0.7 mL/min, which achieves an average of 250 bar when running. Testing at higher standard rates can also be carried out, such as for example up to 1.1 mL/min to stress test the pump core. Higher flow rates may be used if the resistance of the nozzle in the pump core is reduced for product specific applications.

### Power Pack

In this embodiment, the power pack 4 comprises a stable DC power supply. It is preferred that a stable DC power supply is used, in order to reduce the rippling effect observed when using AC/DC electrical supplies. The ripple effect can cause 'noise' in the readings which can unacceptably distort the results. The power pack 4 is connected to the pressure sensor 3 and the data capture device via power cables 12 so as to provide power in use.

### Data Capture Device

A data capture device is required. In this embodiment, the data capture device 5 comprises a National Instruments card NI 9205. Suitable alternatives could be used.

### Computer

Any suitable computing device capable of running software to control the pump and capable of receiving data from the data capture device can be used as the computer 6. In this embodiment, the computer used is chosen so as to be able to run NI DAQExpress software, so as to record data from the NI 9205 card. The computer is connected via cables 11 to the pump 2 and the data capture device 5, so as to receive and send signals to and from these devices as required.

### Tubing/Connectors

In this embodiment, stainless steel capillary tubing and fittings are used as the hydraulic tubing 9, in order to provide robust seals which can take high pressures without leaking. PEEK connections are prone to leaking under the rapid increases of pressure and it is therefore preferred that 0.5 mm ID / 1/16" OD Stainless Steel Capillary Tubing is used, along with Stainless Steel ferrules and nuts.

Although PEEK tubing can be used, this has a lower maximum pressure rating and so it is preferred that stainless steel tubing is used.

Two- and three-way unions are used to connect the hydraulic tubing 9 as required. A three-way union is used at the junction 10, as this allows the pressure sensor to be connected in-line, which enables high frequency sampling of pressure (this is not common in a high standard pump system for commercial use).

### Pressure Sensor

Any suitable pressure sensor 3 that enables high frequency sampling of pressure can be used. In this embodiment, a pressure sensor compatible with the chosen or preferred form of data capture device 5 is used. In the preferred embodiment, a Wika S-20 is used.

### Fixture

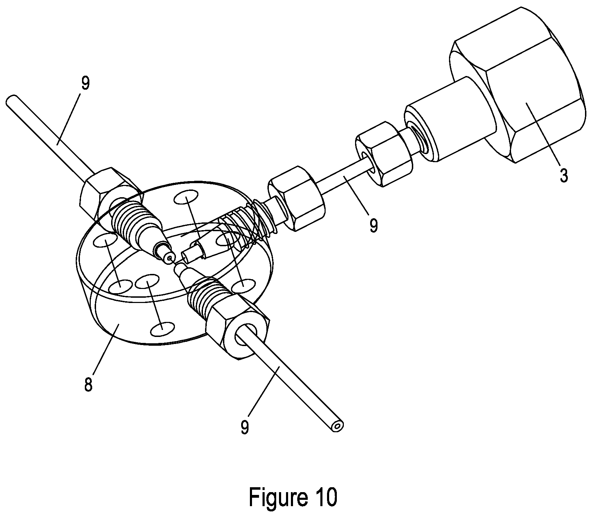

An example of a fixture 8 suitable for testing pump cores is shown in figure 10. In use, a pump core 15 to be tested is connected to the fixture 8, with the fixture fluidically connected to the pump 2 via the hydraulic tubing/connectors 9. The pump 2 pumps a liquid at high pressure to the fixture 8, and then through the core 15 at a given flow rate, and the response from the pressure sensor(s) 3 is used to determine the functionality of the core. Figure 10 shows the preferred form of three-way connection. As noted above, a three-way union is used at the junction 10, as this allows the pressure sensor to be connected in-line. It is preferred that the core 15 is configured so that when the pump core 15 is connected, dead space within the port, where air can be trapped, is minimised.

### METHOD

In this embodiment of the method, the core 15 of a soft mist inhaler is connected to the fixture 8.

The reservoir 7 is filled with HPLC grade ethanol, as this is the preferred form of fluid for testing. However, any other suitable fluid can be used instead.

The pump 2 is run so as to pump the ethanol at a set rate through the device core 15. The pump 2 can be set to pump at a rate of flow that represents the expected average flow rate for the core 15. This rate will be dependent on the specific configuration of the pump core and nozzle. The pump could also be set to pump at a rate of flow that represents the highest anticipated pressure during device actuation.

The pressure sensor measures and reports a voltage to the computer 6 via the data capture device 5. The voltage measured corresponds to the pressure within the core 15. The voltage/pressure is measured over the full pump sequence - ramping up, steady state flow, and ramping down.

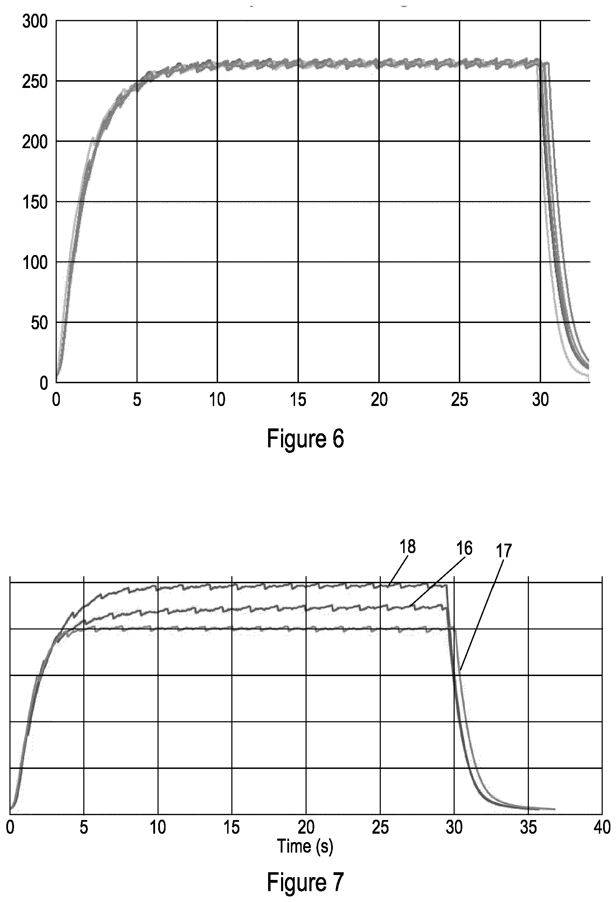

The pressure/voltage is plotted against time. An example of the graph produced, for six test runs at a flow rate of 0.7 mL/min is shown in figure 6. Pressure in bar is shown on the y-axis, and time in seconds is shown on the x-axis.

As can be seen, the plotted pressure v time curve has a first 'ramp up' phase, a 'steady state' phase, and a 'ramp down' phase.

All aspects of the response curve can be analysed as they can provide an indication of performance. The parameters which may be useful in determining device performance include:
- Ramp up rate/angle
- Steady state flow pressure
- Ramp down rate/angle
- Any sudden increases or decreases in pressure.

Gross or subtle differences in these parameters can indicate defects in the nebuliser devices. For example:
- A core that shows a steady high resistance likely indicates a minor, immovable blockage, or possibly that the flow channels are too small (by manufacture).
- A core that shows a steady low resistance likely indicates that the flow channels are too large (by manufacture), or that a leak path is present in the device.
- A test run on a pump core where the curve reaches a high resistance and does not plateau indicates a complete blockage of the pump core.
- A test run where there is a shallow ramp up angle or no levelling out of the pressure measurement likely indicates a leak in the pump core.
- A plot where there is no ramp up indicates a severe leak and complete failure of the pump core.
- A plot where there is a high peak followed by a drop and levelling off of the pressure back to the anticipated level likely shows a blockage which has been cleared during testing, or that a blockage which has resulted in over pressurisation of the pump core has caused a leak path to be created.
- A plot showing a slow ramp down after an acceptable steady state flow likely indicates that the pump core has become partially blocked.
- A plot showing a fast ramp down after an acceptable steady state flow likely indicates that the pump core has ruptured or become damaged at high pressure.

It should be noted that in the embodiment described above, the core 15 of a soft mist inhaler is connected to the fixture 8. That is, a part of a liquid delivery device (the core as a sub-part of the soft mist inhaler) is connected to the pumping system. If required, the part of the liquid delivery device connected to the fixture 8/pumping system could comprise the core plus other parts of the liquid delivery device, up to the whole of the device (excepting those parts that are required to be removed for connection to the pumping system. If this is the case, it can be assumed for the purposes of reading this specification that the whole of the liquid delivery device has been connected). Alternatively, fewer parts can be connected to the pumping system, such as for example just the nozzle assembly (nozzle retainer 115, nozzle 101 and nozzle seal 111).

That is, a liquid delivery device or part thereof is fluidically connected to a pumping system, in order to run the testing method.

### TEST RESULTS

### Normal, Wide, and Narrow-Channel Pump Cores

As noted above, figure 6 shows the plots for six test runs at a flow rate of 0.7 mL/min for a core that is within specification.

In contrast, figure 7 shows three plots, with pressure plotted on the y-axis against time on the x-axis, similarly to figure 6.

Plot 16 on figure 7 is the plot for a nominal or within specification core, added to the graph for the purposes of comparison with plot 17 and plot 18.

Plot 17 shows the plot for a wide-channel pump core. It can be seen that the peak pressure in the 'steady state' phase is lower than for the 'within specification' core of plot 16.

Plot 18 shows the plot for a narrow-channel pump core. It can be seen that the peak pressure in the 'steady state' phase is higher than for the 'within specification' core of plot 16.

These plots demonstrate that pressure testing of this type can be used to determine whether cores and components within the cores have flow channels that have been manufactured within the required tolerances, or if they are outside tolerance.

### Two-Outlet Pump Core

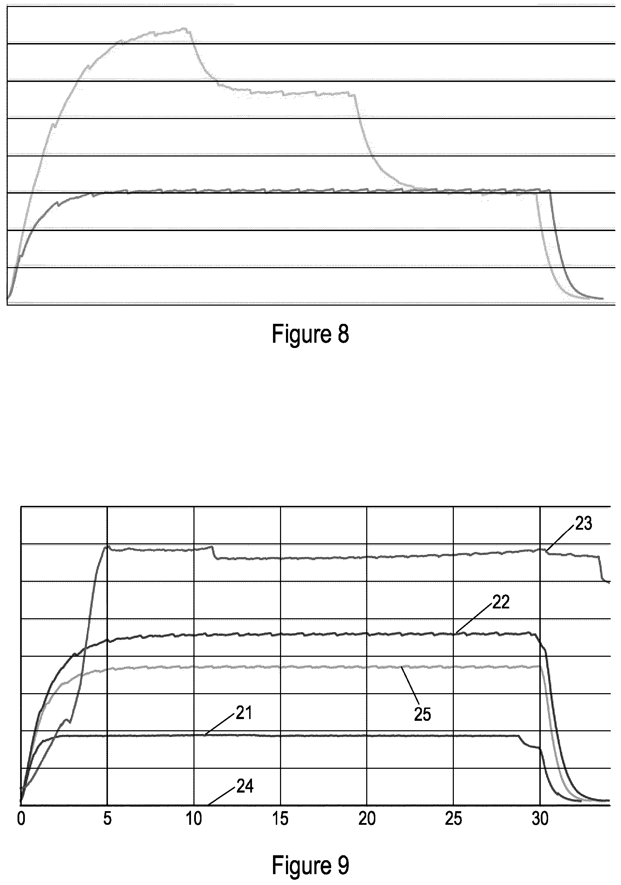

A further example is shown in figure 8. In the graph of figure 8, pressure is plotted on the y-axis against time on the x-axis, similarly to figures 6 and 7.

In this example, two separate test runs were carried out on the same pump core. The pump core used in this test is of the type that has two outlets. In this particular design of pump core, the outlets are relatively small-size, and can therefore easily become blocked during manufacture or assembly.

Plot 19 shows/represents the first test run carried out on the core. Plot 20 shows/represents the second test run carried out on the core. The expected running pressure for this variant of pump core variant is represented by the third horizontal line on the y-axis of the graph.

Plot 19 shows that the pressure in the pump core greatly exceeds the anticipated pressure during the ramp up phase. This indicates that both of the two outlets in the nozzle are blocked (the nozzle is fully blocked). However, it can be seen that once the pressure reaches a maximum peak, the pressure then drops significantly, but not fully down to the anticipated steady state level. This indicates that the blockage on one side of the nozzle has cleared - one of the two outlets is now unblocked. After further running, the pressure drops again, this time to the anticipated pressure level. This indicates that the blockage on the second side of the nozzle has also cleared - both of the two outlets are now unblocked, and the pump core then runs at the anticipated pressure.

As a check, a second test run is carried out. Plot 20 shows/represents the second test run, and it can be seen that the results are as expected, with a ramp up phase to the expected steady state level, a consistent steady state level, and a ramp-down phase as anticipated, following the steady-state phase. This demonstrates that a blocked nozzle can not only be identified but also become unblocked as a result of this test - a defective product is turned into an acceptable product, which assists with increasing manufacturing yield.

### Damaged Seal, Partially Blocked, Inverted and Damaged Nozzle Pump Cores

A further example is shown in figure 9. In the graph of figure 9, pressure is plotted on the y-axis against time on the x-axis, similarly to the previous figures.

In this example, separate test runs were carried out on separate cores, one having a damaged seal (plot 21), one having a partially blocked nozzle (plot 22), one with an inverted nozzle (plot 23), and one that has a damaged component that causes a pressure leak (plot 24). A 'standard' or nominal plot for a core operating within specification is also shown (plot 25).

The pump core with a damaged seal (plot 21) measures a lower pressure than the nominal pressure represented by plot 25, due to the constant leak from the pump core when pressure is applied. As shown in plot 21, the pressure is consistent during the steady state phase. However, it should be noted that this is not always the case with leaking cores, and in some cases the leak may increase as pressure is applied and may not result in a steady state as shown here.

The pump core with a partially blocked nozzle (plot 22) measures a higher pressure than the nominal pressure represented by plot 25, due to the channels through the core effectively being narrowed by the partial blockage.

The pump core with an inverted nozzle (plot 23) shows a very high pressure during the steady state phase in comparison to the nominal pressure represented by plot 25, due to flow not easily passing through the nozzle in its inverted orientation.

The pump core with a damaged component causing a leak (plot 24) shows no resistance (pressure) due to the severe leak. The pressure measured is essentially zero, and plot 24 substantially follows the zero line of the y-axis.

### ADVANTAGES

As noted above, it has been found that a good approximation of operability performance can be made by measuring the resistance of a nebuliser "pump core" as a fluid such as Ethanol is pumped through it, using the method described above. This test has a quick cycle time, and requires less management of ethanol/aerosolised matter due the test not requiring a firing chamber to measure and characterise spray quality. The equipment required is relatively low cost, and the test as a whole is less costly to implement than other types of known tests.

This test also has the advantage that multiple testing stations can be run in parallel with one another, each testing station carrying out testing according to the apparatus and method of the present invention. This multiple testing in parallel helps to prevent bottlenecks on the assembly lines, and also has the advantage of decreasing the time required for product release.

This test method can be used to filter out sub-standard products and may be used as a product release test at the 'end of line'. Alternatively, this test can be applied early before final assembly/end of line, so as to avoid or reduce scrapping of additional components.

Additional advantages of a pressure test of this type include:
- Higher pressures than would be used in normal use can be applied to the core in order to provide confidence in the robustness of the assembly (noting that a too-high testing pressure may also cause issues such as for example seal damage from excess pressure, with this then being a potential cause for failure in a device).
- The pressure during a test run can be ramped up gently so that impact cycling of the device is minimised. The benefits of this include reduced O ring wear, reduced stress on internal parts, and reduced formation of debris that can occur due to moving parts contacting seals.
- The spray duration can be estimated from the pressure curve in systems where flow rate is not the controlled parameter, e.g. in a sprung piston configuration.
- Debris which could be blocking the core may be flushed out during testing, and therefore faulty or error-prone devices that would otherwise be rejected can be passed as working devices.
- Testing the pump core alone further up the assembly chain reduces the incidence of scrap of other componentry that is included in the finalised device. This additional componentry is added further down the assembly line, prior to an end-of-line test for the finished device. If an end-of-line test shows that the device is sub-standard, then the entire device is scrapped, and not just the core. Testing just the core and rejecting defective cores cuts down on the incidence of scrap of other componentry.

## Claims

1. **A method for testing the functionality of a liquid delivery device,** comprising the steps of:
(i) fluidically connecting a liquid delivery device (or part thereof) to a pumping system;
(ii) connecting the pumping system to a fluid reservoir;
(iii) running the pumping system so that fluid from the reservoir is forced through the liquid delivery device (or part thereof) under pressure;
(iv) measuring the pressure of the fluid as the fluid passes into the liquid delivery device (or part thereof), the pressure measured over the full pump sequence;
(v) plotting the pressure measurement at at least one point of the pump sequence and comparing the plotted pressure with the test results for a nominal or within specification liquid delivery device (or part thereof).

2. A method for testing the functionality of a liquid delivery device as claimed in claim 1 wherein in step (v), the pressure measure measurements are plotted over substantially the full pump sequence.

3. A method for testing the functionality of a liquid delivery device as claimed in claim 1 or claim 2 wherein in the step of connecting the pump to a fluid reservoir, the pump is connected to a reservoir containing ethanol.

4. A method for testing the functionality of a liquid delivery device as claimed in any one of claims 1 to 3 wherein in the step of running the pump, the pump is run so as to deliver fluid at a rate of substantially up to 1.1 mL/min through the liquid delivery device.

5. A method for testing the functionality of a liquid delivery device as claimed in any one of claims 1 to 3 wherein in the step of running the pump, the pump is run so as to deliver fluid at a rate of substantially 0.7 mL/min through the liquid delivery device.

6. **An apparatus for testing the functionality of a liquid delivery device,** comprising:
a high pressure pumping system;
at least one pressure sensor connected the pumping system and configured to measure the pressure or resistance of the liquid delivery device;
a computer adapted to connect to and control the pump, and to receive data from at least one pressure sensor and to plot the measured pressure against time.
